# EUROPEAN PATENT APPLICATION

(11) **EP 4 796 067 A1**
(43) Date of publication of application: **26.08.2026**
(21) Application number: 24879017.2
(22) Date of filing: 16.10.2024
(51) Int. Cl.: A61B 34/37

(54) **MASTER HAND OPERATION CONTROL SYSTEM AND METHOD, MASTER-END DEVICE, AND SURGICAL ROBOT**

(30) Priority: 18.10.2023 CN 202311353259
(71) Applicant: Shanghai Microport Medbot (Group) Co., Ltd., Shanghai 201203 (CN)
(72) Inventor: ZHANG, Honghai, Shanghai 201203 (CN); GONG, Qingzheng, Shanghai 201203 (CN); JIANG, Lei, Shanghai 201203 (CN); ZHENG, Ayong, Shanghai 201203 (CN); WANG, Jiayin, Shanghai 201203 (CN)
(74) Representative: Patentship Patentanwaltsgesellschaft mbH
(86) International application number: PCT/CN2024/125153
(87) International publication number: WO 2025/082375

(57) **Abstract**

Provided are a system and method for controlling operation of a master manipulator, a master-end device and a surgical robot. The system includes a movable button (610) disposed on a terminal joint (117) of the master manipulator (110) and a controller (620) communicatively connected to the movable button (610). The movable button (610) is movable relative to the terminal joint (117). The movable button (610) is configured to be operated by an operator to trigger an action-triggering command and transfer the action-triggering command triggered by the operator to the controller (620). The action-triggering command includes at least one of a command for triggering surgical procedure confirmation, a command for triggering recovery from an error condition and a command for triggering switching to use of the movable button for clamping control. The controller (620) is configured to receive the action-triggering command and cause an action associated with the action-triggering command to be taken. This system allows various actions to be taken without interrupting a surgical procedure, ensuring continuity of the procedure.

## Description

### TECHNICAL FIELD

The present application relates to the field of surgical robotics, and more particularly to a system and method for controlling operation of a master manipulator, a master-end device and a surgical robot.

### BACKGROUND

Surgical robots are designed to perform complex surgical procedures with high precision in a minimally invasive manner. A surgical robot includes a master-end device and a slave device. The master-end device is operated by a surgeon to generate and transmit necessary signals, and the slave device receives the signals and physically operates on a patient under the control of the received signals. The master-end device includes a master manipulator, which can be manipulated by the surgeon to control a robotic arm in the slave device and a surgical instrument attached to the robotic arm at its terminal end to move in a corresponding way.

During surgical use of existing endoscopic surgical robots, recovery from an error condition is generally accomplished by interacting with a user interface (UI), and procedure confirmation is typically accomplished by interacting with the slave device, in contrast to the fact that the surgeon must continually operate the master manipulator throughout the procedure. Therefore, the existing robots could not ensure continuity of a surgical procedure. Moreover, in the event of Hall sensor failure occurring in such existing surgical robots, the master manipulator may become incapable of opening/closing control over an end effector of the surgical instrument.

It should be noted that the information disclosed in this Background section is merely intended to provide a better understanding of the general context of the present invention and should not be taken as an acknowledgement or any form of admission that the information forms part of the common general knowledge of those skilled in the art.

### SUMMARY

It is an object of the present application to provide a system and method for controlling operation of a master manipulator, a master-end device and a surgical robot, which allows various actions to be taken during a surgical procedure, without interrupting the procedure, thereby ensuring its continuity.

To this end, disclosed herein is a system for controlling operation of a master manipulator, which includes a movable button disposed on a terminal joint of the master manipulator and a controller communicatively connected to the movable button. The movable button is movable relative to the terminal joint.

The movable button is configured to be operated by an operator to trigger an action-triggering command and transfer the action-triggering command triggered by the operator to the controller. The action-triggering command includes at least one of a command for triggering surgical procedure confirmation, a command for triggering recovery from an error condition and a command for triggering switching to use of the movable button for clamping control.

The controller is configured to receive the action-triggering command and cause an action associated with the action-triggering command to be taken.

Optionally, the movable button may include a button body and a sensor coupled to the button body, wherein the sensor is configured to detect information about a travel length of the movable button and transfer an electrical signal carrying the information to the controller.

Optionally, the controller may be further configured to allow the operator to define a trigger event for the action-triggering command.

Optionally, the trigger event for the action-triggering command may include at least one of a combination of the movable button and at least one of acoustic activation, a pedal switch, a manipulator switch and an eye-movement monitor being triggered, the movable button being triggered for a predetermined number of times within a first predetermined period of time, the movable button being held at a maximum travel limit for a second predetermined period of time and movable buttons on different master manipulators being triggered in a predetermined order.

Optionally, the controller may be further configured to determine whether the movable button is triggered based on its travel length and a period of time for which it is held at a location corresponding to the travel length.

Optionally, the controller may be further configured, during the use of the movable button for clamping control, to control an opening angle at a terminal end of a surgical instrument on a slave device based on information about a travel length of the movable button.

Optionally, the controller may be further configured, in the case of a single trigger event being configured for a plurality of action-triggering commands, based on predefined priority levels for the action-triggering commands, to cause an action associated with one of the action-triggering commands, which has the highest one of the priority levels, to be taken.

To the above end, disclosed herein is also a method for controlling operation of a master manipulator. The master manipulator is provided with a movable button on its terminal joint. The movable button is movable relative to the terminal joint and configured to be operated by an operator to trigger an action-triggering command. The action-triggering command includes at least one of a command for triggering surgical procedure confirmation, a command for triggering recovery from an error condition and a command for triggering switching to use of the movable button for clamping control. The method includes:
receiving the action-triggering command triggered by the operator; and
causing an action associated with the action-triggering command to be taken.

To the above end, disclosed herein is also a master-end device including a system for controlling operation of a master manipulator as defined above.

To the above end, disclosed herein is also a surgical robot including a master-end device as defined above.

The system, method, master-end device and surgical robot disclosed herein offer the following benefits over the prior art:
The system includes a movable button disposed on a terminal joint of a master manipulator and a controller communicatively connected to the movable button. The movable button is movable relative to the terminal joint. The movable button is configured to be operated by an operator to trigger an action-triggering command and transfer the action-triggering command triggered by the operator to the controller. The action-triggering command includes at least one of a command for triggering surgical procedure confirmation, a command for triggering recovery from an error condition and a command for triggering switching to use of the movable button for clamping control. The controller is configured to receive the action-triggering command and cause an action associated with the action-triggering command to be taken. With this arrangement, an operator is allowed to take various actions by operating the movable button during a surgical procedure, including surgical procedure confirmation, recovery from an error condition and switching to use of the movable button for clamping control, without interrupting the surgical procedure, ensuring continuity of the surgical procedure and making a surgical robot easier to use.

Since the method, master-end device and surgical robot disclosed herein share the same inventive concept as the system disclosed herein and therefore have at least all its benefits. For more details of these benefits, reference is made to the above description in connection with the system, and further detailed description thereof is omitted herein for the sake of brevity.

### BRIEF DESCRIPTION OF THE DRAWINGS

Fig. 1 schematically illustrates a scenario to which a surgical robot according to an embodiment disclosed herein is applicable.
Fig. 2 is a schematic illustration of a master-end device according to an embodiment disclosed herein.
Fig. 3 is a schematic illustration of a master manipulator according to an embodiment disclosed herein.
Fig. 4 shows a schematic block diagram of a system for controlling operation of a master manipulator according to an embodiment disclosed herein.
Fig. 5 schematically illustrates how a movable button is coupled to a master manipulator in accordance with an embodiment disclosed herein.
Fig. 6 shows a flowchart of a detection process performed by a circuit associated with a movable button according to an embodiment disclosed herein.
Fig. 7 is a schematic illustration of a surgical instrument according to an embodiment disclosed herein.
Fig. 8 shows a flowchart of a process for calculating a Hall-sensor opening angle, which is an opening angle defined at a terminal end of a surgical instrument, according to an embodiment disclosed herein.
Fig. 9 schematically illustrates how a movable button is coupled to a master manipulator in accordance with another embodiment disclosed herein.
Fig. 10 shows a flowchart of a detection process performed by a circuit associated with a movable button according to another embodiment disclosed herein.
Fig. 11 shows a flowchart of a process for calibrating a detection range of a movable button according to an embodiment disclosed herein.
Fig. 12 schematically illustrates trigger events for action-triggering commands according to an embodiment disclosed herein.
Fig. 13 shows a flowchart of a process for rapid surgical procedure confirmation by manipulating a movable button according to an embodiment disclosed herein.
Fig. 14 schematically illustrates what is displayed on a user interface of a master-end device under an error condition.
Fig. 15 shows a flowchart of a process for rapid recovery from an error condition by manipulating a movable button according to an embodiment disclosed herein.
Fig. 16 shows a flowchart of a process for switching to the use of a movable button for clamping control according to an embodiment disclosed herein.
Fig. 17 schematically illustrates priority levels of action-triggering commands according to an embodiment disclosed herein.
Fig. 18 shows a flowchart of an algorithm for preventing false triggering of a movable button according to an embodiment disclosed herein.
Fig. 19 shows a flowchart of a method for controlling operation of a master manipulator according to an embodiment disclosed herein.
Fig. 20 shows a schematic block diagram of an electronic device according to an embodiment disclosed herein.

### DETAILED DESCRIPTION

Systems and methods for controlling operation of a master manipulator, master-end devices, surgical robots and electronic devices according to specific embodiments disclosed herein will be described in greater detail below with reference to the accompanying drawings. From the following description, advantages and features of the present invention will become more apparent. Note that the figures are provided in a very simplified form not necessarily drawn to exact scale for the only purpose of helping to explain the disclosed embodiments in a more convenient and clearer way. It would be recognized that architectural, proportional, dimensional and other details in the figures are presented only for the purpose of facilitating, in conjunction with the disclosure herein, the understanding and reading of those familiar with the art, rather than being intended to limit conditions under which the present invention can be implemented. Therefore, any and all architectural modifications, proportional variations or dimensional changes that achieve the same or similar benefits and objects as the present invention are considered to fall within the scope of the teachings herein.

In principle, the present application seeks to provide a system and method for controlling operation of a master manipulator, a master-end device and a surgical robot, which allow various actions to be taken during a surgical procedure without interrupting the procedure, thus ensuring its continuity. It should be noted and will be appreciated by those skilled in the art that, as used herein, the term "terminal" refers to an end closer to a lesion when describing a slave device, but to an end closer to an operator when describing a master-end device. Also, it should be noted and will be appreciated by those skilled in the art that electronic devices proposed herein can be used, possibly in the form of hardware devices capable of running various operating systems, in systems for controlling operation of a master manipulator proposed herein.

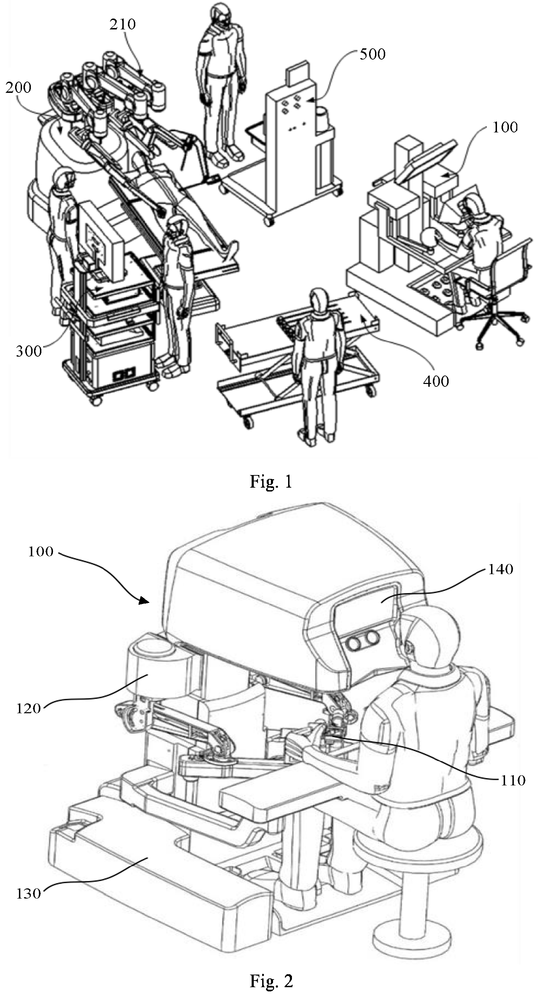

For ease of understanding, before systems and methods for controlling operation of a master manipulator, master-end devices and surgical robots proposed herein are detailed, a scenario in which the surgical robots can be suitably used is briefed below. Reference is now made to Figs. 1 and 2. Fig. 1 schematically illustrates a scenario to which a surgical robot according to an embodiment disclosed herein is applicable. Fig. 2 is a schematic illustration of a master-end device according to an embodiment disclosed herein. As shown in Figs. 1 and 2, a surgical robot proposed herein includes a master-end device 100 and a slave device 200. The master-end device 100 includes a master manipulator 110, and the slave device 200 includes at least one robotic arm 210, to which a surgical instrument 220 and an endoscope (not shown) can be separately attached. An operator (e.g., a surgeon) can operate the master manipulator 110 in the master-end device 100 to teleoperatively perform a minimally invasive surgical procedure on a patient lying in a hospital bed. The master manipulator 110 is in a master/slave control relationship with the robotic arm 210 and the surgical instrument 220, meaning that the robotic arm 210 and the surgical instrument 220 follow movements of the master manipulator 110 caused by the operator's hand operations during surgery. The master manipulator 110 also receives information about reaction forces exerted by the patient's tissue and organs upon the surgical instrument 220 and feeds this information back to the operator's hands, allowing him/her to get more intuitive feelings about the operations. The master-end device 100 includes a display 140, which is communicatively connected to the endoscope attached to the robotic arm 210 in the slave device 200, in order to receive and display images captured by the endoscope. Based on the images displayed on the display 140 of the master-end device 100, the operator can control movements of the robotic arm 210 and the surgical instrument 220 by operating the master manipulator 110. The endoscope and the surgical instrument 220 may be inserted into the patient's body through separate incisions made in the body and held at appropriate locations relative to a target lesion.

Continued reference is made to Fig. 2. As shown in Fig. 2, the master-end device 100 may include an adjustment member 120, a console 130, two master manipulators 110 and a display 140. The two master manipulators 110 detect information about the operator's hand movements using their terminal joints 117, which is taken as input to the system for motion control. The console 130 serves as a base support for mounting of other components. The console 130 may be equipped with casters (not shown), which allow the console to be moved or immobilized, as desired. The console 130 may further include a pedal switch (not shown), which is capable of detecting on-off control signals from the operator. The adjustment member 120 may be able to electrically adjust the positions of the master manipulators 110, the display 140, operator armrests and other components. That is, it may have the ability to adjust ergonomic parameters. The display 140 can provide the operator with reliable visual information for supporting his/her surgical maneuvers. During surgery, the operator may be seated in front of the master-end device 100, which may be deployed outside of a sterile area, and control the surgical instrument 220 and the endoscope by operating the master manipulators 110 to perform various surgical maneuvers, as may be required by a surgical procedure planned for the patient. In addition, the operator may utilize the pedal switch to control some actions, such as providing input to electroresection, electrocoagulation and similar tasks.

With continued reference to Fig. 2, the surgical robot further includes an imaging cart 300 and an instrument cart 400. Images captured by the endoscope inside the patient's body (in particular, containing information about tissue and organs, the surgical instrument 220, blood vessels, body fluids, etc.) can be transferred to the imaging cart 300 for display. The instrument cart 400 is adapted for storage of surgical instruments 220. For some surgical procedures, the surgical robot may further include certain auxiliary components, which are necessary for use in such procedures, such as a ventilator and an anesthesia machine 500. Those skilled in the art may select and configure these auxiliary components based on the common general knowledge in the art, and further description thereof is omitted herein.

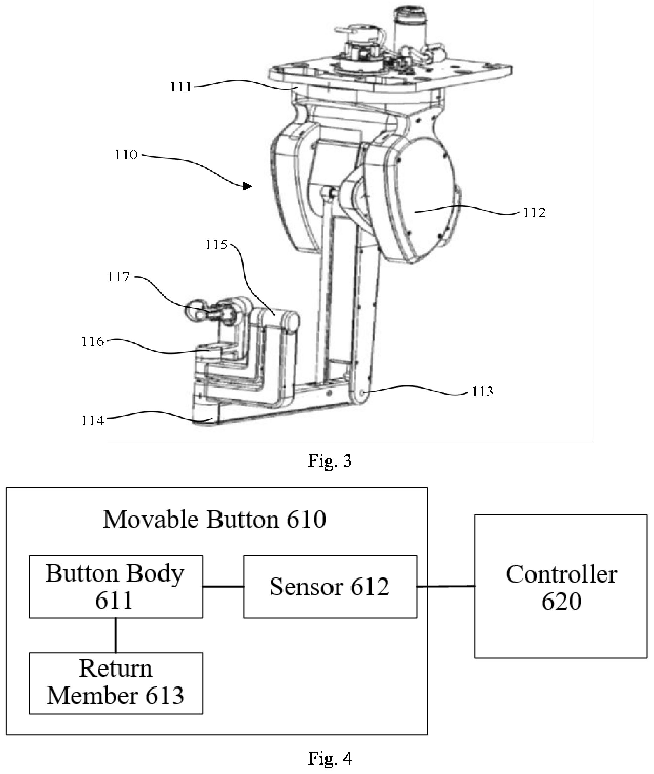

Reference is additionally made to Fig. 3, which is a schematic illustration of the master manipulator 110 according to an embodiment disclosed herein. As shown in Fig. 3, the master manipulator 110 includes a first joint 111, a second joint 112, a third joint 113, a fourth joint 114, a fifth joint 115, a sixth joint 116 and a terminal joint 117, which are connected in sequence. The first, second and third joints 111, 112, 113 are positioning joints. The fourth joint 114 is a redundant follower joint, and the fifth, sixth and terminal joints 115, 116, 117 are orientation joints. A movement of any positioning joint, such as the first, second or third joints 111, 112, 113 will cause a change in position of a terminal end of the master manipulator 110. Axes of the fifth, sixth and terminal joints 115, 116, 117 intersect at a single point corresponding to a Cartesian position of the terminal end. Movements of the fifth, sixth and terminal joints 115, 116, 117 do not alter the position of the terminal end of the master manipulator 110, but result in changes in the orientation of the master manipulator 110 at the terminal end.

Reference is additionally made to Fig. 4, which shows a schematic block diagram of a system for controlling operation of a master manipulator according to an embodiment disclosed herein. As shown in Fig. 4, the system includes a movable button 610 provided on the terminal joint 117 of the master manipulator 110 and a controller 620 communicatively connected to the movable button 610. The movable button 610 can be moved relative to the terminal joint 117, and is configured to be operated by an operator to trigger an action-triggering command and transfer the action-triggering command triggered by the operator to the controller 620. The action-triggering command includes at least one of a command for triggering surgical procedure confirmation, a command for triggering recovery from an error condition and a command for triggering switching to use of the movable button for clamping control. The controller 620 is configured to receive the action-triggering command and cause an action associated with the action-triggering command to be taken. Thus, through manipulating the movable button 610, the operator is enabled to take various actions during a surgical procedure, including surgical procedure confirmation, recovery from an error condition and switching to use of the movable button for clamping control, without interrupting the procedure, thereby ensuring continuity of the surgical procedure making the surgical robot easier to use.

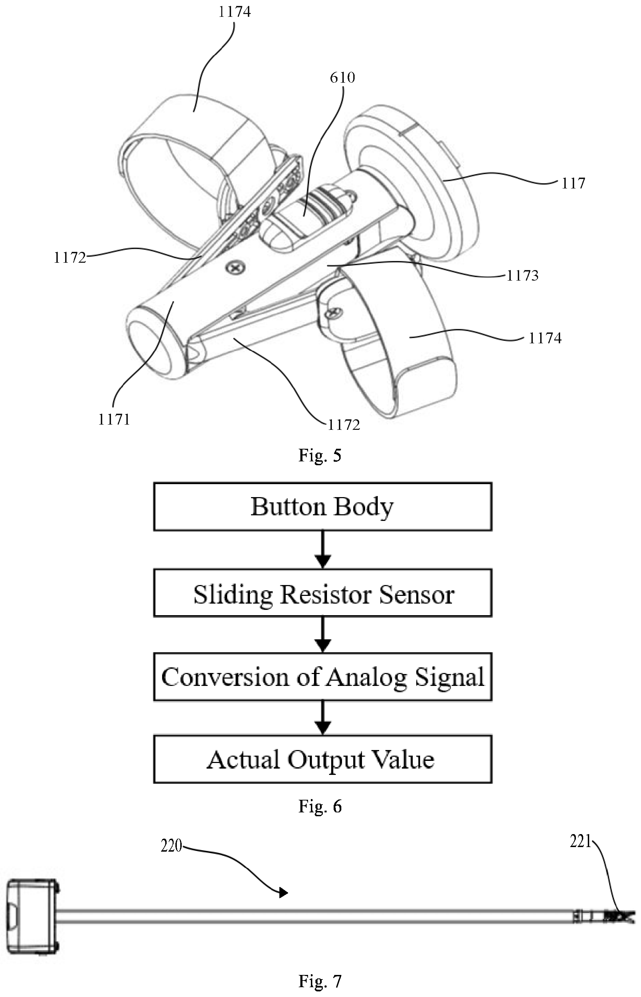

Specifically, after the surgical robot is started up, the operator may check whether the slave device 200 has been prepared to commence the procedure. If so, he/she may directly issue a command for triggering procedure confirmation by manipulating the movable button 610 to confirm the commencement of the surgical procedure. Once the slave device 200 is activated in response to such manipulation of the movable button 610, the operator may manipulate the master manipulator 110 to control movement of the robotic arm 210 in the slave device 200 and the surgical instrument 220 and/or endoscope at the terminal end of the robotic arm 210 to ensure continuous performance of the procedure. In the event of the surgical robot system encountering an error condition during the surgical procedure, after the error condition is cleared, the operator may issue a command for triggering recovery of the surgical robot system from the error condition by manipulating the movable button 610, thereby avoiding interrupting the procedure and ensuring its continuity. If it is found during the procedure that a Hall sensor 1173 fails (see Fig. 5), or that an inappropriate Hall sensor 1173 is used, the surgical instrument 220 may become incapable of successful or accurate clamping, the operator may issue, by manipulating the movable button 610, a command for triggering switching to use of the movable button 610 for clamping control. In response, the system can be rapidly switched from opening/closing control relying on the Hall sensor to clamping control by the movable button 610, leading to additionally improved continuity of the surgical procedure. It should be noted and will be appreciated by those skilled in the art that, in practical applications, an operator may manipulate the movable button 610 to issue action-triggering commands for triggering other actions than surgical procedure confirmation, recovery from an error condition and switching to use of the movable button 610 for clamping control. Such action-triggering commands may be configured based on practical considerations, and the present application is not limited to any particular action-triggering command.

In some exemplary embodiments, the movable button 610 includes a button body 611 and a sensor 612 coupled to the button body 611. The sensor 612 is configured to detect information about a travel length of the movable button 610 and transmit an electrical signal carrying the information to the controller 620, which can then obtain the information from the electrical signal. This provides a good basis for subsequently determining, by the controller 620, when receiving an action-triggering command, what action is associated with the command. It should be noted and will be appreciated by those skilled in the art that the present application is not limited to any particular type of sensor 612. The sensor 612 may be a sliding resistor 612 or a pressure sensor. Of course, it may also be any other suitable sensor known to those skilled in the art that is capable of detecting a travel length of the movable button 610.

Additionally, the movable button 610 further includes a return member abutting against the button body 611. The return member is adapted to return the movable button 610 to its rest position, increasing ease of operation. It should be noted and will be appreciated by those skilled in the art that the return member may be, but is not limited to being, implemented as a return spring 613.

In a preferred embodiment, two movable buttons 610 of the type discussed above are respectively provided on upper and lower sides of the terminal joint 117 in the master manipulator 110. This can additionally increase ease of operation.

Reference is additionally made to Fig. 5, which schematically illustrates how the movable button 610 is coupled to the master manipulator 110 in accordance with an embodiment disclosed herein. As shown in Fig. 5, in some exemplary embodiments, the movable button 610 is a sliding button. Accordingly, an operator may trigger the movable button 610 by sliding it along the axis of the terminal joint 117 in the master manipulator 110.

Additionally, when the movable button 610 is implemented as a sliding button, the sensor 612 may be implemented as a sliding resistor 612. Specifically, reference is now made to Fig. 6, a flowchart of a detection process performed by a circuit associated with the movable button 610 according to an embodiment disclosed herein. As shown in Fig. 6, in the case of the movable button 610 being implemented as a sliding button, after it is slid by an operator, a signal indicating its travel length is responsively transmitted through the button body 611 to the sensor 612, which then converts the signal into a corresponding analog resistance signal and provides it to the controller 620. The controller 620 then in turn converts the analog resistance signal into an actual output value of the movable button 610 (which indicates its travel length). It should be noted and will be appreciated by those skilled in the art that in the case of the sensor 612 being implemented as a sliding resistor 612, the actually output value of the movable button 610 (which indicates the travel length) may be obtained as a corresponding resistance value.

Continued reference is made to Fig. 5. As shown in Fig. 5, in some exemplary embodiments, the terminal joint 117 of the master manipulator 110 includes a support rod 1171 and two clamping sheets 1172 disposed on opposite sides of the support rod 1171. Both clamping sheets 1172 are inclined relative to the support rod 1171, and can be caused to move toward or away from each other. The support rod 1171 is provided with a Hall sensor 1173, and magnets are provided on the respective sides of the respective clamping sheets 1172 that face the support rod 1171, enabling the Hall sensor 1173 to detect an opening angle defined between the two clamping sheets 1172 (referred to hereinafter as a Hall-sensor opening angle), namely, an opening angle defined at a terminal end 221 of the surgical instrument 220 on the slave device 200 (see Fig. 7, a schematic illustration of the surgical instrument 220 according to an embodiment disclosed herein).

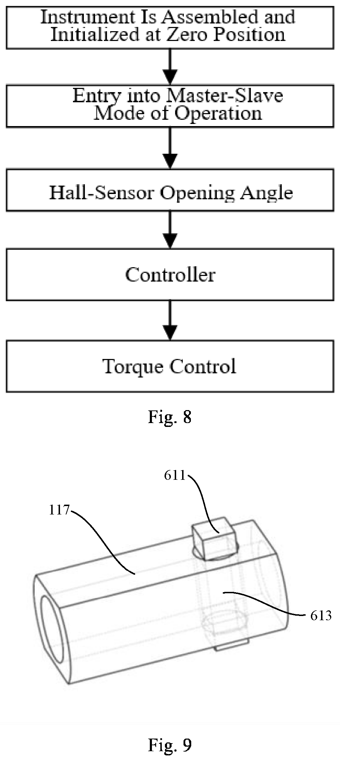

Reference is additionally made to Fig. 8, which shows a flowchart of a process for calculating a Hall-sensor opening angle, i.e., an opening angle at the terminal end 221 of the surgical instrument according to an embodiment disclosed herein. As shown in Fig. 8, after being assembled, the surgical instrument 220 may be initialized at its zero position. After entering a master-slave mode of operation, the controller 620 may regulate an output torque of a motor for causing opening/closing at the terminal end 221 of the surgical instrument 220 based on Hall-sensor opening angle feedback from the Hall sensor 1173, thereby achieving effective opening/closing control at the terminal end 221 of the surgical instrument 220.

Continued reference is made to Fig. 5. As shown in Fig. 5, each of the clamping sheets 1172 is further provided with a finger sleeve 1174. An operator may insert his/her fingers into the respective finger sleeves 1174 to control an opening angle between the two clamping sheets 1172, resulting in a further increase in ease of operation.

Reference is additionally made to Fig. 9, which schematically illustrates how the movable button 610 is coupled to the master manipulator 110 in accordance with another embodiment disclosed herein. As shown in Fig. 9, in some other exemplary embodiments, the movable button 610 is provided in the form of a press button. In these embodiments, an operator may trigger the movable button 610 by pressing the movable button 610 along the normal of the terminal joint 117 of the master manipulator 110.

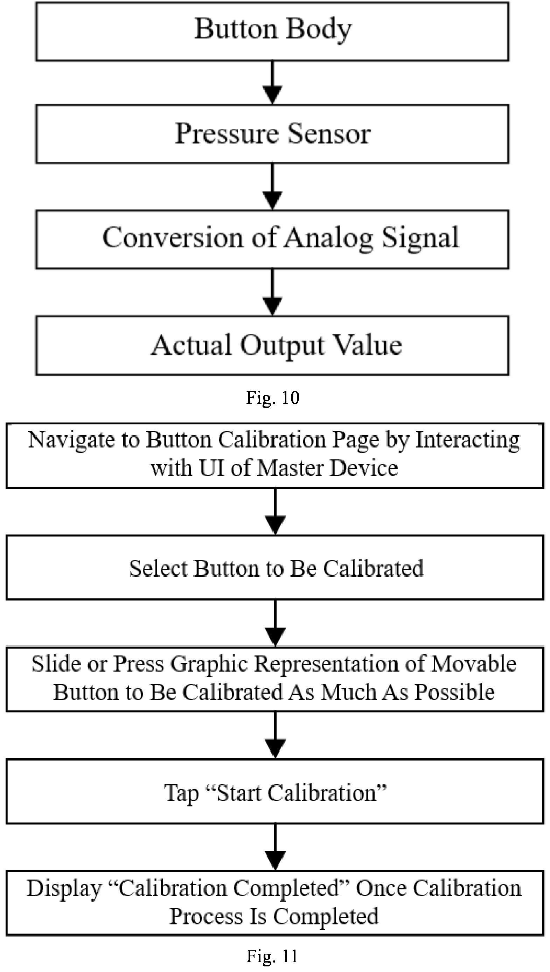

Further, in the case of the movable button 610 being implemented as a press button, the sensor 612 may be provided as a pressure sensor. Specifically, reference is made to Fig. 10, a flowchart of a detection process performed by a circuit associated with the movable button 610 according to another embodiment disclosed herein. As shown in Fig. 10, in the case of the movable button 610 being implemented as a press button, after it is pressed by an operator, a signal indicating its travel length is responsively transmitted through the button body 611 to the pressure sensor, which then converts the signal into a corresponding analog pressure signal and provides it to the controller 620. The controller 620 then in turn converts the analog pressure signal into an actual output value of the movable button 610 (which indicates the travel length). It should be noted and will be appreciated by those skilled in the art that in the case of the sensor 612 being implemented as a pressure sensor, the actually output value of the movable button 610 (which indicates the travel length) may be obtained as a corresponding pressure value.

After the movable button 610 is used for a period of time, a shift may occur in the detection range of the sensor 612, necessitating a detection range recalibration. Specifically, if the sensor 612 in the movable button 610 is implemented as a sliding variable resistor, the detection range calibration may be carried out essentially to reestablish a correct ratio of a maximum possible travel length for the button body 611 to the maximum resistance of the sliding variable resistor. If the sensor 612 in the movable button 610 is implemented as a pressure sensor, the detection range calibration may be carried out essentially to reestablish a correct ratio of the maximum possible travel length for the button body 611 to the highest pressure that the pressure sensor is designed to measure. Reference is additionally made to Fig. 11, which shows a flowchart of a process for calibrating the detection range of the movable button 610 according to an embodiment disclosed herein. As shown in Fig. 11, an operator may interact with a user interface (UI) of the master-end device 100 to navigate to a button calibration page, and then select the movable button 610 to be calibrated (i.e., "Select Button to Be Calibrated"). After that, he/she may slide or press a graphic representation of the movable button 610 to be calibrated as much as possible (to cause the movable button 610 to reach a maximum travel limit) and then tap "Start Calibration" to obtain an output electrical signal from the sensor 612 at the maximum travel limit. This marks the end of the calibration process. Once the calibration process is completed, a "Calibration Completed" notification may be displayed on the UI.

In some exemplary embodiments, the controller 620 is further configured, during use of the movable button 610 for clamping control, to control an opening angle at the terminal end 221 of the surgical instrument 220 on the slave device 200 based on information about a travel length of the movable button 610. Specifically, a calibration process may be performed in advance to map travel lengths of the movable button 610 to corresponding opening angles at the terminal end 221 and used as a basis for calculating, by the controller 620, a desired opening angle at the terminal end 221 according to a real-time travel length measurement of the movable button 610, and then a desired output torque of the opening/closing motor according to the calculated opening angle at the terminal end 221. In this way, effective opening/closing control can be achieved at the terminal end 221 of the surgical instrument 220.

In some exemplary embodiments, the controller 620 is further configured to allow an operator to define trigger events for action-triggering commands. Thought configuring a trigger event for each action-triggering command, such action-triggering commands can be more easily identified, facilitating correct triggering of the associated actions.

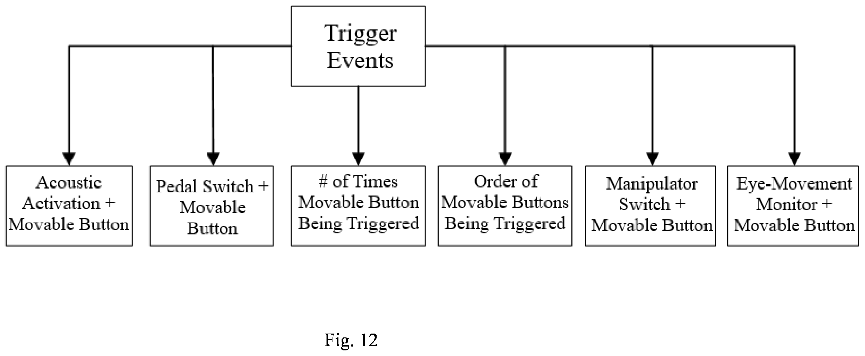

Reference is additionally made to Fig. 12, which schematically illustrates trigger events for action-triggering commands according to an embodiment disclosed herein. As shown in Fig. 12, in some exemplary embodiments, a trigger event for an action-triggering command may include at least one of a combination of the movable button 610 and at least one of acoustic activation, the pedal switch, a manipulator switch and an eye-movement monitor being triggered, the movable button 610 being triggered for a predetermined number of times within a first predetermined period of time, the movable button 610 being held at a maximum travel limit for a second predetermined period of time and movable buttons 610 on different master manipulators 110 being triggered in a predetermined order. In particular, an operator may configure trigger events for various action-triggering commands based on practical considerations, and the present application is not limited to any trigger event for any action-triggering command.

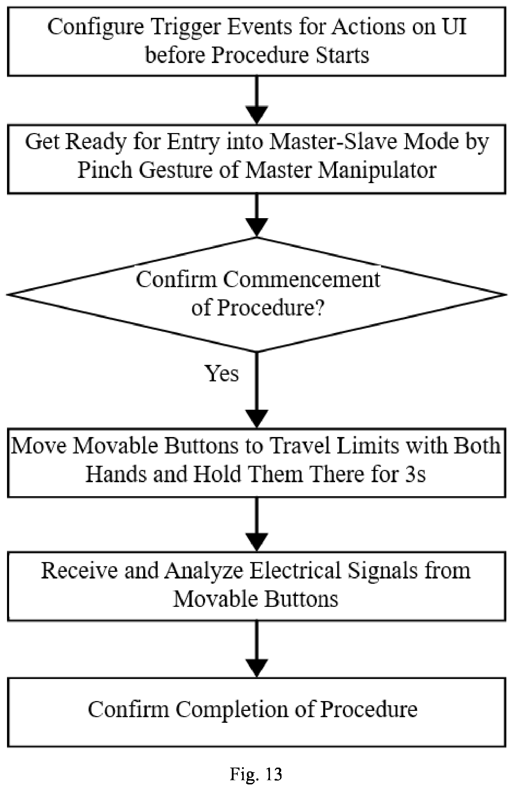

Reference is additionally made to Fig. 13, which shows a flowchart of a process for rapid surgical procedure confirmation by manipulating the movable button 610 according to an embodiment disclosed herein. As shown in Fig. 13, in a master-slave mode of operation during a surgical procedure, an operator may issue a command for triggering surgical procedure confirmation by manipulating the movable button 610 on the terminal joint 117 in the master manipulator 110 (e.g., by moving movable buttons 610 on respective two master manipulators 110 to their maximum travel limits and holding them there for 3 seconds). In this way, surgical procedure confirmation can be achieved in a continuous style. Once the slave device 200 is activated and prepared for the surgical procedure by manipulating the movable button 610, the operator can manipulate the master manipulator 110 to control movement of the robotic arm 210 in the slave device 200 and the surgical instrument 220 and/or endoscope at the terminal end of the robotic arm 210 to ensure continuous performance of the procedure. It should be noted and will be appreciated by those skilled in the art that, as shown in Fig. 13, the controller 620 may analyze a received electrical signal indicating an action-triggering command to identify an action associated with the command.

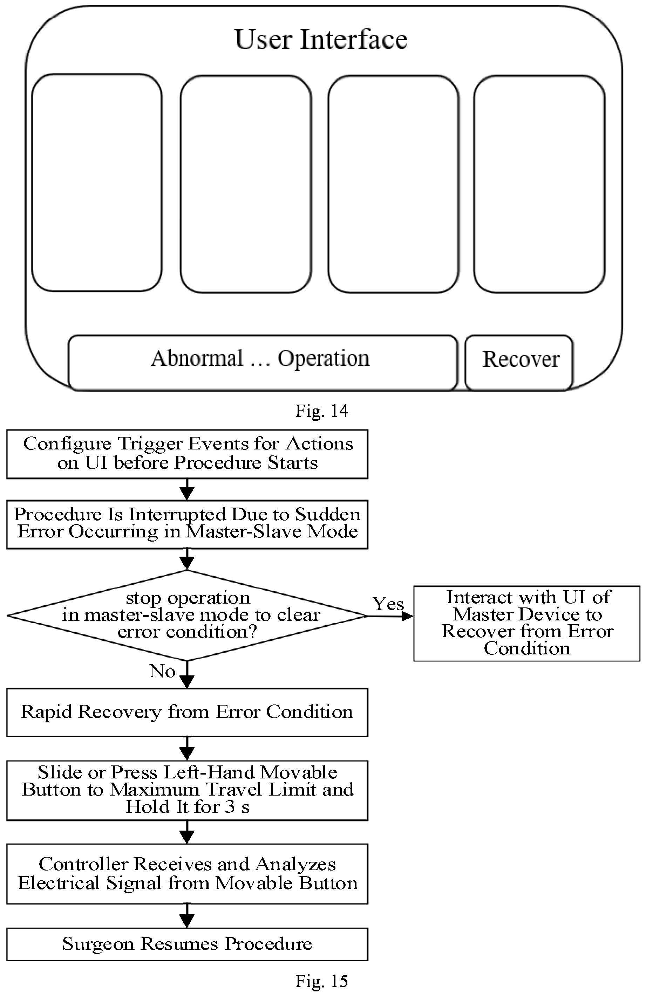

Reference is additionally made to Figs. 14 and 15. Fig. 14 schematically illustrates what is displayed on the UI of the master-end device 100 under an error condition. Fig. 15 shows a flowchart of a process for rapid recovery from an error condition by manipulating the movable button 610 according to an embodiment disclosed herein. As shown in Figs. 14 and 15, in the event of an error condition being reported in the UI on the display 140 of the master-end device 100, an operator may issue a command for triggering recovery from the error condition by manipulating the movable button 610 on the terminal joint 117 in the master manipulator 110 (e.g., by moving the movable button 610 on a left-hand one of the master manipulators 110 to its maximum travel limit and holding it for 3 seconds). In this way, recovery (of a master-slave mode of operation) from an error condition during a surgical procedure can be achieved rapidly in a continuous fashion. In contrast, conventionally, such recovery would necessitate tapping the UI on the display 140 of the master-end device 100. Thus, the present application ensures continuity of a surgical procedure by avoiding interruptions that may be necessary in such scenarios.

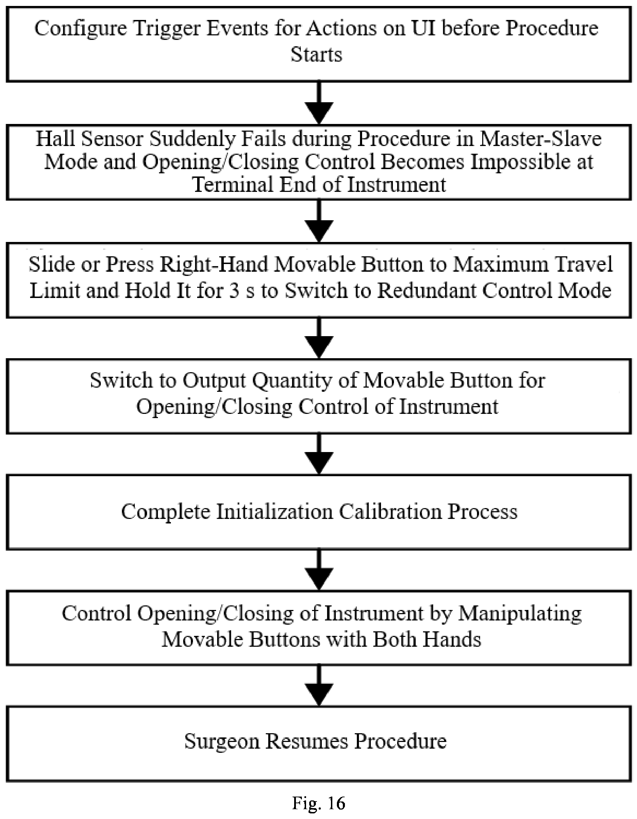

Reference is additionally made to Fig. 16, which shows a flowchart of a process for switching to use of the movable button 610 for clamping control according to an embodiment disclosed herein. As shown in Fig. 16, in the event of the Hall sensor 1173 on the terminal joint 117 of the master manipulator 110 failing, an operator may issue a command for triggering switching to use of the movable button 610 for clamping control by manipulating the movable button 610 on the terminal joint 117 in the master manipulator 110 (e.g., by moving the movable button 610 on a right-hand one of the master manipulators 110 to its maximum travel limit and holding it for 3 seconds). In this way, once the Hall sensor 1173 fails in a master-slave mode of operation during a surgical procedure, an output quantity of the movable button 610 (i.e., a travel length of the movable button 610, which is, for example, represented by an output resistance or pressure value of the movable button 610) can be used, after being subjected to an initialization calibration process, in place of an output quantity of the Hall sensor 1173 (i.e., a Hall-sensor opening angle), to provide a redundant (spare) control mechanism for the terminal end 221 of the surgical instrument 220 on the slave device 200. Such clamping control over the terminal end 221 based on the control quality from the movable button 610 can ensure continuity of the surgical procedure. Further, the output quantity of the movable button 610 can also be used to control rotation of the endoscope on the slave device 200 and spinning of the surgical instrument 220. It should be noted and will be appreciated by those skilled in the art that, as shown in Fig. 16, the initialization calibration process is performed to establish a mapping between values of the output quantity of the movable button 610 and opening angles at the terminal end 221.

In some exemplary embodiments, the controller 620 is further configured, in the case of a single trigger event being configured for a plurality of action-triggering commands, based on predefined priority levels for the action-triggering commands, to cause an action associated with the one of the action-triggering commands that has the highest priority level to be taken. A single trigger event may be configured for a plurality of action-triggering commands, and priority levels may be predefined for the respective action-triggering commands. Thus, when the trigger event occurs, an action associated with the action-triggering command having the highest priority level may be taken. This obviates the otherwise need for an operator to frequently memorize trigger events for respective action-triggering commands, reducing operating complexity and additionally increasing ease of operation.

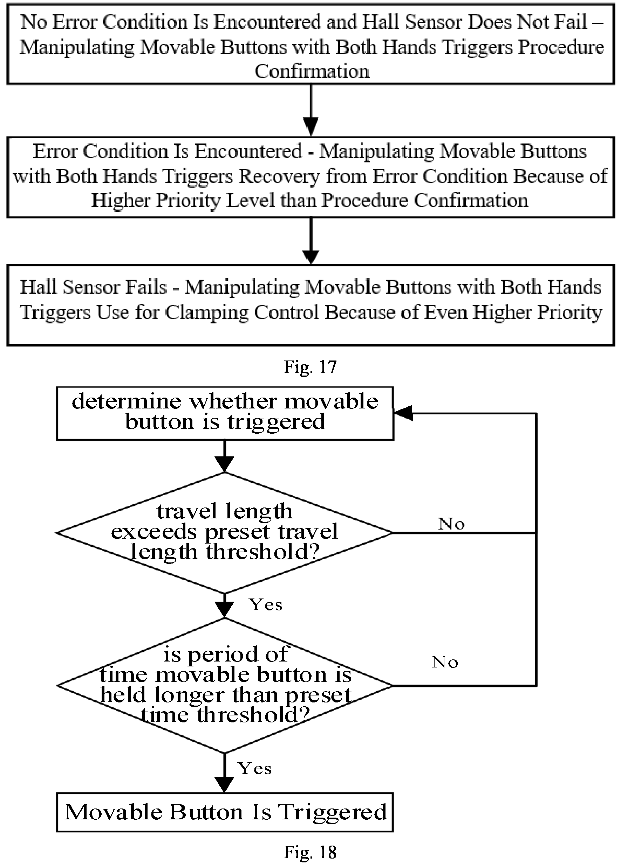

Specifically, reference is made to Fig. 17, which schematically illustrates priority levels of action-triggering commands according to an embodiment disclosed herein. As shown in Fig. 17, in some exemplary embodiments, a priority level for a command for triggering surgical procedure confirmation may be lower than that for a command for triggering recovery from an error condition, which may be in turn lower than that for a command for triggering switching to use of the movable button 610 for clamping control. Accordingly, if there is no error condition being reported and the Hall sensor 1173 does not fail, an operator may trigger a command for triggering surgical procedure confirmation by sliding or pressing the movable buttons 610 with both hands. If an error condition is encountered and the Hall sensor 1173 does not fail, when an operator slides or presses the movable buttons 610 with both hands, a command for triggering recovery from the error condition will be triggered because of its higher priority level than a command for triggering surgical procedure confirmation. If the Hall sensor 1173 fails, when an operator slides or presses the movable buttons 610 with both hands, a command for triggering switching to use of the movable button 610 for clamping control will be triggered because of its highest priority level.

In some exemplary embodiments, the controller 620 is further configured to determine whether the movable button 610 is triggered based on its travel length and a period of time for which it is held at a location corresponding to the travel length. This can reject interference from external and internal signals with the triggering of the movable button 610, ensuring accurate signal acquisition and providing an effective mechanism for preventing false triggering of the movable button 610.

Specifically, reference is made to Fig. 18, which shows a flowchart of an algorithm for preventing false triggering of the movable button 610 according to an embodiment disclosed herein. As shown in Fig. 18, in order to detect whether the movable button 610 is triggered, it may be first determined whether its travel length exceeds a preset travel length threshold. If the determination is positive, it may be further determined whether a period of time for which the movable button 610 is held at a location corresponding to the travel length is longer than a preset time threshold. If the determination is positive, it is determined that the movable button 610 is triggered.

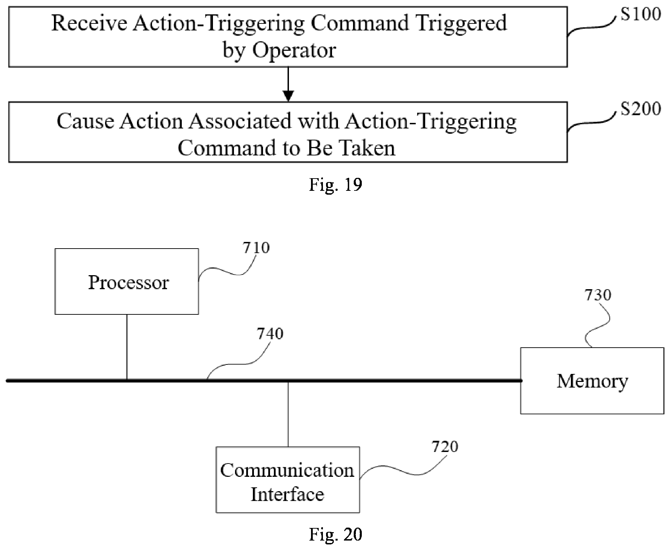

Also disclosed herein is a method for controlling operation of a master manipulator. The master manipulator 110 is provided with a movable button 610 on its terminal joint 116, which is movable relative to the terminal joint 117. The movable button 610 is configured to be operated by an operator to trigger an action-triggering command. The action-triggering command includes at least one of a command for triggering surgical procedure confirmation, a command for triggering recovery from an error condition and a command for triggering switching to use of the movable button for clamping control. Reference is additionally made to Fig. 19, which shows a flowchart of the method according to an embodiment disclosed herein. As shown in Fig. 19, the method includes the steps of:
S100) receiving the action-triggering command triggered by the operator; and
S200) causing an action associated with the action-triggering command to be taken.

In this way, the method allows various actions to be taken during a surgical procedure, without interrupting the procedure, thereby ensuring its continuity and increasing ease of use of a surgical robot.

In some exemplary embodiments, the method further includes:
defining a trigger event for the action-triggering command by the operator.

In some exemplary embodiments, the trigger event for the action-triggering command includes at least one of a combination of the movable button 610 and at least one of acoustic activation, the pedal switch, a manipulator switch and an eye-movement monitor being triggered, the movable button 610 being triggered for a predetermined number of times within a first predetermined period of time, the movable button 610 being held at a maximum travel limit for a second predetermined period of time and movable buttons 610 on different master manipulators 110 being triggered in a predetermined order.

In some exemplary embodiments, the method further includes:
determining whether the movable button 610 is triggered based on its travel length and a period of time for which it is held at a location corresponding to the travel length.

In some exemplary embodiments, the action-triggering command includes at least one of a command for triggering surgical procedure confirmation, a command for triggering recovery from an error condition and a command for triggering switching to use of the movable button 610 for clamping control.

In some exemplary embodiments, the method further includes:
during the use of the movable button 610 for clamping control, controlling an opening angle at a terminal end 221 of a surgical instrument 220 on a slave device 200 based on information about a travel length of the movable button 610.

In some exemplary embodiments, in the case of a single trigger event being configured for a plurality of action-triggering commands, the method further includes:
based on predefined priority levels for the action-triggering commands, causing an action associated with one of the action-triggering commands, which has the highest one of the priority levels, to be taken.

Also disclosed herein is a master-end device 100 including a system for controlling operation of a master manipulator as discussed above. Since the master-end device 100 includes the system, it has at least all the benefits of the system. For more details of these benefits, reference is made to the above description in connection with the system, and further detailed description thereof is omitted herein for the sake of brevity. It should be noted and will be appreciated by those skilled in the art that, for more details of this master-end device 100, reference is made to the above description in connection with the master-end device 100, and further detailed description thereof is omitted herein for the sake of brevity.

Also disclosed herein is a surgical robot including a master-end device 100 as discussed above. Since the surgical robot includes the master-end device 100, which in turn includes a system for controlling operation of a master manipulator as discussed above, it also has at least all the benefits of the system. For more details of these benefits, reference is made to the above description in connection with the system, and further detailed description thereof is omitted herein for the sake of brevity. It should be noted and will be appreciated by those skilled in the art that, for more details of this surgical robot, reference is made to the above description in connection with the surgical robot, and further detailed description thereof is omitted herein for the sake of brevity.

Also disclosed herein is an electronic device. Reference is now made to Fig. 20, which shows a schematic block diagram of the electronic device according to an embodiment disclosed herein. As shown in Fig. 20, the electronic device includes a processor 710 and memory 730 storing therein a computer program which, when executed by the processor 710, implements a method for controlling operation of a master manipulator as discussed above. Since the electronic device shares the same inventive concept as the method, it has at least all the benefits of the method. For more details of these benefits, reference is made to the above description in connection with the method, and further detailed description thereof is omitted herein for the sake of brevity.

As shown in Fig. 20, the electronic device further includes a communication interface 720 and a communication bus 740. Communication among the processor 710, the communication interface 720 and the memory 730 is accomplished through the communication bus 740. The communication bus 740 may be, among others, a peripheral component interconnect (PCI) bus or extended industry standard architecture (EISA) bus. The communication bus 740 may be an address bus, data bus, control bus or other bus. Although it is represented by only one bold line in the figure for ease of illustration, this does not mean that there is only one bus, or only one type of bus. The communication interface 720 is used to enable communication of the electronic device with other devices.

In the present embodiment, the processor 710 may be a central processing unit (CPU) or other general-purpose processor, a digital signal processor (DSP), an application specific integrated circuit (ASIC), a field-programmable gate array (FPGA) or other programmable logic device, a discrete gate or transistor-transistor logic device, a discrete hardware component, or the like. The general-purpose processor may be a microprocessor or any other conventional processor. The processor 710 may serve as a control center of the electronic device, and the various components in the electronic device may be interconnected with various interfaces and lines.

The memory 730 can be used to store a computer program, and the processor 710 can perform various functions of the electronic device by running or executing the computer program stored in the memory 730 and retrieving data stored in the memory 730. The memory 730 may include non-volatile and/or volatile memory. Non-volatile memory may include read-only memory (ROM), programmable ROM (PROM), electrically programmable ROM (EPROM), electrically erasable programmable ROM (EEPROM) or flash memory. Volatile memory may include random-access memory (RAM) or external cache memory. For illustration rather than limitation, RAM is available in many forms such as static RAM (SRAM), dynamic RAM (DRAM), synchronous DRAM (SDRAM), double-data-rate SDRAM (DDRSDRAM), enhanced SDRAM (ESDRAM), Synchlink DRAM (SLDRAM), Rambus direct RAM (RDRAM), Direct Rambus DRAM (DRDRAM), Rambus DRAM(RDRAM), etc.

As discussed above, disclosed herein are a system and method for controlling operation of a master manipulator, a master-end device 100, a surgical robot and an electronic device, which offer the following benefits over the prior art:
An operator is allowed to trigger commands for triggering various actions during a surgical procedure, including surgical procedure confirmation, recovery from an error condition and switching to use of the movable button 610 for clamping control, by manipulating a movable button 610 provided on a terminal joint 117 of the master manipulator 110, without interrupting the surgical procedure, thereby ensuring continuity of the surgical procedure and making a surgical robot easier to use.

It is to be noted that the devices and methods in the embodiments disclosed herein may also be implemented in other ways and the device embodiments described above are merely illustrative. For example, the flowcharts and block diagrams in the figures illustrate the architecture, functionality and operation of possible implementations of devices, methods, and computer program products according to various embodiments disclosed herein. In this regard, each block in the flowcharts or block diagrams may represent a module, segment, or portion of instructions, which includes one or more executable instructions for implementing the specified logical function(s). It is to be also noted that, in some alternative implementations, the functions noted in the blocks may occur out of the order noted in the figures. For example, two blocks shown in succession may, in fact, be executed substantially concurrently, or the blocks may sometimes be executed in the reverse order, depending upon the functionality involved. It is to be further noted that each block of the block diagrams and/or flowchart illustrations, and combinations of blocks in the block diagrams and/or flowchart illustrations, can be implemented by special purpose hardware-based systems that perform the specified functions or acts or carry out combinations of special purpose hardware and computer instructions. Further, the various functional modules in the embodiments disclosed herein may be integrated into a discrete component, or provided as separate modules. Alternatively, two or more such modules may be integrated into a discrete component.

It should be noted that the description presented above is merely that of a few preferred embodiments of the present invention and is not intended to limit the scope thereof in any sense. Any and all changes and modifications made by those of ordinary skill in the art in light of the above teachings fall within the scope as defined in the appended claims. Apparently, those skilled in the art can make various modifications and variations to the present invention without departing from the spirit and scope thereof. Accordingly, the invention is intended to embrace all such modifications and variations if they fall within the scope of the appended claims and equivalents thereof.

## Claims

1. A system for controlling operation of a master manipulator, comprising a movable button disposed on a terminal joint of the master manipulator and a controller in communication connection with the movable button, the movable button being capable of moving relative to the terminal joint,
the movable button configured to be operated by an operator to trigger an action-triggering command and transfer the action-triggering command triggered by the operator to the controller, the action-triggering command comprising at least one of a command for triggering surgical procedure confirmation, a command for triggering recovery from an error condition and a command for triggering switching to use of the movable button for clamping control,
the controller configured to receive the action-triggering command and cause an action associated with the action-triggering command to be taken.

2. The system for controlling operation of the master manipulator according to claim 1, wherein the movable button comprises a button body and a sensor coupled to the button body, the sensor configured to detect travel information of the movable button and transfer an electrical signal carrying the travel information of the movable button to the controller.

3. The system for controlling operation of the master manipulator according to claim 1, wherein the controller is further configured to allow the operator to define a trigger event for the action-triggering command.

4. The system for controlling operation of the master manipulator according to claim 3, wherein the trigger event for the action-triggering command comprises at least one of a combination of the movable button and at least one of acoustic activation, a pedal switch, a manipulator switch and an eye-movement monitor being triggered, the movable button being triggered for a predetermined number of times within a first predetermined period of time, the movable button being held at a maximum travel limit for a second predetermined period of time and movable buttons on different master manipulators being triggered in a predetermined order.

5. The system for controlling operation of the master manipulator according to claim 1, wherein the controller is further configured to determine whether the movable button is triggered based on a travel length of the movable button and a period of time for which it is held at a location corresponding to the travel length.

6. The system for controlling operation of the master manipulator according to claim 1, wherein the controller is further configured, during the use of the movable button for clamping control, to control an opening angle at a terminal end of a surgical instrument on a slave device based on travel information of the movable button.

7. The system for controlling operation of the master manipulator according to claim 1, wherein the controller is further configured, in the case of a single trigger event being configured for a plurality of action-triggering commands, based on predefined priority levels for the action-triggering commands, to cause an action associated with one of the action-triggering commands, which has the highest one of the priority levels, to be taken.

8. A method for controlling operation of a master manipulator, **characterized in that** a movable button is arranged on a terminal joint of the master manipulator, the movable button being movable relative to the terminal joint, the movable button configured to be operated by an operator to trigger an action-triggering command, the action-triggering command comprising at least one of a command for triggering surgical procedure confirmation, a command for triggering recovery from an error condition and a command for triggering switching to use of the movable button for clamping control, the method comprising:
receiving the action-triggering command triggered by the operator; and
causing an action associated with the action-triggering command to be taken.

9. A master-end device, comprising the system for controlling operation of the master manipulator as defined in any one of claims 1 to 7.

10. A surgical robot, comprising the master-end device as defined in claim 9.
